Europäisches Patentamt

European Patent Office

Office européen des brevets

(19)

(11) **EP 1 514 944 A1**

(12) **EUROPEAN PATENT APPLICATION**

(43) Date of publication:
**16.03.2005 Bulletin 2005/11**

(51) Int Cl.7: **C12Q 1/48**, G01N 33/566

(21) Application number: **03020429.1**

(22) Date of filing: **11.09.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **Aventis Pharma Deutschland GmbH**
**65929 Frankfurt am Main (DE)**

(72) Inventors:
• **Kannt, Aimo, Dr.**
**60598 Frankfurt (DE)**

• **Pommereau, Antje**
**67697 Otterberg (DE)**
• **Thuering, Harald, Dr.**
**60596 Frankfurt (DE)**
• **Huber, Jochen, Dr.**
**67133 Maxdorf (DE)**
• **Oppermann, Martin, Prof., Dr.**
**37075 Göttingen (DE)**

(54) **High-throughput-Assay with high sensitivity for measuring of the activity of beta-adrenergic receptor kinase and for determining the impact of test substances on such activity**

(57)     The invention refers to an assay for determining the activity of a G-Protein-Coupled Receptor specific protein kinase by means of an amplified luminescent proximity homogenous assay.

EP 1 514 944 A1

SEQUENCE LISTING

<110> Aventis Pharma Deutschland GmbH

<120> High-Throughput-Assay with high sensitivity for
      measuring of the activity of ß-adrenergic receptor
      kinase and for determining of the impact of test
      substances on such activity

<130> DEAV2003/0065

<140>
<141>

<160> 2

<170> PatentIn Ver. 2.1

<210> 1
<211> 22
<212> PRT
<213> Homo sapiens

<400> 1
Ala Pro Glu Arg Ala Ser Ser Val Tyr Thr Arg Ser Thr Gly Glu Gln
 1               5                  10                  15

Glu Ile Ser Val Gly Leu
            20

<210> 2
<211> 689
<212> PRT
<213> Homo sapiens

<400> 2
Met Ala Asp Leu Glu Ala Val Leu Ala Asp Val Ser Tyr Leu Met Ala
 1               5                  10                  15

Met Glu Lys Ser Lys Ala Thr Pro Ala Ala Arg Ala Ser Lys Lys Ile
            20                  25                  30

Leu Leu Pro Glu Pro Ser Ile Arg Ser Val Met Gln Lys Tyr Leu Glu
            35                  40                  45

Asp Arg Gly Glu Val Thr Phe Glu Lys Ile Phe Ser Gln Lys Leu Gly

```
                50              55                 60

        Tyr Leu Leu Phe Arg Asp Phe Cys Leu Asn His Leu Glu Glu Ala Arg
         65              70              75                  80

        Pro Leu Val Glu Phe Tyr Glu Glu Ile Lys Lys Tyr Glu Lys Leu Glu
                     85              90                  95

        Thr Glu Glu Glu Arg Val Ala Arg Ser Arg Glu Ile Phe Asp Ser Tyr
                    100             105             110

        Ile Met Lys Glu Leu Leu Ala Cys Ser His Pro Phe Ser Lys Ser Ala
                    115             120             125

        Thr Glu His Val Gln Gly His Leu Gly Lys Lys Gln Val Pro Pro Asp
                    130             135             140

        Leu Phe Gln Pro Tyr Ile Glu Glu Ile Cys Gln Asn Leu Arg Gly Asp
        145             150             155             160

        Val Phe Gln Lys Phe Ile Glu Ser Asp Lys Phe Thr Arg Phe Cys Gln
                    165             170             175

        Trp Lys Asn Val Glu Leu Asn Ile His Leu Thr Met Asn Asp Phe Ser
                    180             185             190

        Val His Arg Ile Ile Gly Arg Gly Gly Phe Gly Glu Val Tyr Gly Cys
                    195             200             205

        Arg Lys Arg Asp Thr Gly Lys Met Tyr Ala Met Lys Cys Leu Asp Lys
                    210             215             220

        Lys Arg Ile Lys Met Lys Gln Gly Glu Thr Leu Ala Leu Asn Glu Arg
        225             230             235             240

        Ile Met Leu Ser Leu Val Ser Thr Gly Asp Cys Pro Phe Ile Val Cys
                    245             250             255

        Met Ser Tyr Ala Phe His Thr Pro Asp Lys Leu Ser Phe Ile Leu Asp
                    260             265             270

        Leu Met Asn Gly Gly Asp Leu His Tyr His Leu Ser Gln His Gly Val
                    275             280             285

        Phe Ser Glu Ala Asp Met Arg Phe Tyr Ala Ala Glu Ile Ile Leu Gly
                    290             295             300

        Leu Glu His Met His Asn Arg Phe Val Val Tyr Arg Asp Leu Lys Pro
```

305 310 315 320

Ala Asn Ile Leu Leu Asp Glu His Gly His Val Arg Ile Ser Asp Leu
325 330 335

Gly Leu Ala Cys Asp Phe Ser Lys Lys Lys Pro His Ala Ser Val Gly
340 345 350

Thr His Gly Tyr Met Ala Pro Glu Val Leu Gln Lys Gly Val Ala Tyr
355 360 365

Asp Ser Ser Ala Asp Trp Phe Ser Leu Gly Cys Met Leu Phe Lys Leu
370 375 380

Leu Arg Gly His Ser Pro Phe Arg Gln His Lys Thr Lys Asp Lys His
385 390 395 400

Glu Ile Asp Arg Met Thr Leu Thr Met Ala Val Glu Leu Pro Asp Ser
405 410 415

Phe Ser Pro Glu Leu His Ser Leu Leu Glu Gly Leu Leu Gln Arg Asp
420 425 430

Val Asn Arg Arg Leu Gly Cys Leu Gly Arg Gly Ala Gln Glu Val Lys
435 440 445

Glu Ser Pro Phe Phe Arg Ser Leu Asp Trp Gln Met Val Phe Leu Gln
450 455 460

Arg Tyr Pro Pro Pro Leu Ile Pro Pro Arg Gly Glu Val Asn Ala Ala
465 470 475 480

Asp Ala Phe Asp Ile Gly Ser Phe Asp Glu Glu Asp Thr Lys Gly Ile
485 490 495

Lys Leu Leu Asp Ser Asp Gln Glu Leu Tyr Arg Asn Phe Pro Leu Thr
500 505 510

Ile Ser Glu Arg Trp Gln Gln Glu Val Ala Glu Thr Val Phe Asp Thr
515 520 525

Ile Asn Ala Glu Thr Asp Arg Leu Glu Ala Arg Lys Lys Ala Lys Asn
530 535 540

Lys Gln Leu Gly His Glu Glu Asp Tyr Ala Leu Gly Lys Asp Cys Ile
545 550 555 560

Met His Gly Tyr Met Ser Lys Met Gly Asn Pro Phe Leu Thr Gln Trp

```
                565                     570                     575

    Gln Arg Arg Tyr Phe Tyr Leu Phe Pro Asn Arg Leu Glu Trp Arg Gly
                580                     585                 590

    Glu Gly Glu Ala Pro Gln Ser Leu Leu Thr Met Glu Glu Ile Gln Ser
            595                 600                 605

    Val Glu Glu Thr Gln Ile Lys Glu Arg Lys Cys Leu Leu Leu Lys Ile
        610                 615                 620

    Arg Gly Gly Lys Gln Phe Ile Leu Gln Cys Asp Ser Asp Pro Glu Leu
    625                 630                 635                 640

    Val Gln Trp Lys Lys Glu Leu Arg Asp Ala Tyr Arg Glu Ala Gln Gln
                645                 650                 655

    Leu Val Gln Arg Val Pro Lys Met Lys Asn Lys Pro Arg Ser Pro Val
                660                 665                 670

    Val Glu Leu Ser Lys Val Pro Leu Val Gln Arg Gly Ser Ala Asn Gly
            675                 680                 685

    Leu
```

**Description**

High-Throughput-Assay with high sensitivity for measuring of the activity of ß-adrenergic receptor kinase and for determining the impact of test substances on such activity.

The invention refers to a GPCR specific protein kinase assay by means of head linked substrate molecules.

G-protein-coupled receptors (GPCRs) are a very large protein family with thousands of known members. GPCRs share limited amino acid sequence homology and a similar protein topology with an N-terminal extracellular segment, seven hydrophobic membrane spanning $\alpha$-helics and a C-terminal cytoplasmic region. Increased activity of the human heart is mediated by β-adrenergic receptors (β-AR), which are GPCRs responding to adrenergic sympathetic stimulation. Decreased cardiac activity is mediated by the cholinergic parasympathetic system working through muscarinic acetylcholine GPCRs. Cardiac β-AR consists of both the $\beta_1$ and $\beta_2$ subtypes, and in the human heart, as in most mammals, the $\beta_1$ is predominant. The two proteins are rather similar in protein sequence, though the $\beta_1$ is significantly larger due to a larger N-terminal extracellular region and a larger C3 cytoplasmic loop.

These β-AR interact with heterotrimeric guanine nucleotide binding regulatory proteins, usually known as trimeric G-proteins. Trimeric G-proteins consist of an $\alpha$, $\beta$ and y subunit, each represented by several different genes in humans. Six mammalian cDNAs encoding members of the GRK (G Protein Specific Receptor Kinase) subfamily of serine/threonine kinases (EC.2.7.1-) have been identified to date: GRK1 (rhodopsin kinase); GRK2 (ß-adrenergic receptor kinase-1); GRK3 (ß-adrenergic receptor kinase-2); GRK4 ; GRK5; and GRK6. With the exception of GRK1 which is found almost exclusively in retina and GRK4 which is expressed at significant levels only in testes, GRKs are ubiquitously expressed. GRK4 is the only member of this family shown to undergo alternative splicing. Four splice variants exist, with the alternatively spliced exons occurring in the amino and carboxyl termini.

Evolutionary Conservation among GRKs isolated from nematodes, insects, and mammals emphasizes their biological importance. GPRK1 and wj283.2 encode, respectively, Drosophila and Caenorhabditid elegans proteins with 62% and 51 % homology to bovine GRK2. Similary, GPRK2 and f19c6.1 encode Drosophila and C. elegans proteins with 50-53% homology to bovine GRK5. Among the six mammalian GRKs, the amino acid sequence similarity is 53-93%, with GRK1 and GRK2 being the most divergent.

GRKs are most closely related to the PKC and cAMP-dependent protein kinase families (PKAs). Based on sequence and functional similarities, the GRK family has been divided into three subfamilies: (a] GRK1, (b] GRK2 or β-adrenergic receptor kinase (ß-ARK); (GRK2 and GRK3), and (c] GRK4 (GRK4, GRK5, and GRK6). Members of the GRK2 subfamily share approximately 84% sequence similarity, and members of the GRK4 subfamily share approximately 70% sequence similarity.

In the failing human heart, cardiac β-adrenergic receptors are stimulated by an increased release of catecholamines from the sympathetic nerves to maintain cardiac output and blood pressure. This increase in β-adrenergic receptor agonists leads to a downregulation of the receptor (and thus to a lower receptor density) and a receptor desensitization mediated by overexpression of βARK. Thus, treatment with βARK inhibitors may be of benefit for the preservation of myocardial β-adrenergic receptor signaling and may delay the development of heart failure after myocardial infarction.

Assays for testing GRK are known from the state of the art. For example in "Haga et al., 1998, GPT-binding-protein-coupled receptor kinase 2 (GRC2) binds and phosphorylates tubulin, Eur. J. Biochem. 255, 363-368" an assay for GRK2 has been described by means of radioactively labeled substrate molecules.

The AlphaScreen technology is widely used for testing of different types of enzymes. AlphaScreen Assays of different specificity are commercially available e.g. from Perkin-Elmer.

AlphaScreen is a bead based non-radioactive Amplified Luminescent Proximity Homogeneous Assay. When a biological interaction brings the beads together, a cascade of chemical reactions act to produce a greatly amplified signal. On laser excitation, a photosensitizer in the Donor bead converts ambient oxygen to a more excited singlet state. The singlet state oxygen molecules diffuse across to react with a thioxene derivative in the Acceptor bead, generating chemiluminescence at 370 nm that further activates fluorophores contained in the same bead. The fluorophores subsequently emit light at 520-620 nm.

In the absence of a specific biological interaction, the single state oxygen molecules produced by the Donor bead go undetected without the close proximity of the Acceptor bead. As a result no signal is produced.

AlphaScreen provides a highly versatile, sensitive, time-resolved, homogeneous and miniaturizable means to efficiently perform assay development and HTS resulting in higher throughput at lower costs.

The lifetime of singlet oxygen in solution is about 4 $\mu$s which corresponds to a diffusion distance of approximately 200nm. Thus, a signal is only generated when donor and acceptor beads are in close proximity to each other.

In the ßARK inhibition experiment, biotinylated CCR5 peptide (Seq. ID No. 1) is phosphorylated by the kinase. The phosphorylated serine is recognized by a specific antibody which, in turn, is bound to protein A coupled acceptor beads. The biotinylated N-terminus of the CCR5 peptide is coupled to streptavidin-coated donor heads. Thus, in the absence of inhibitors an AlphaScreen signal is detected that decreases when the enzymatic activity in inhibited.

The assay of the invention in particular the test of βARK activity by means of the AlphaScreen technology design is new. Compared to the state of the art determination of the βARK activity is much more sensitive since the change of substrate phosphorylation is detectable within the nM-range.

Furthermore the assay is homogenous which means that additional washing steps or immobilization of reactants are easily avoidable since the reaction is performed by means of solid phases. The assay is suitable for miniaturiarisation which allows the use in high throuput screening by laboratory robots. A further advantage is that the substrate does not need to be modified by covalent binding to fluorescence dyes.

The invention comprises in one embodiment a

Method for detecting the phosphorylation of a substrate molecule by a protein kinase wherein

a] at least one protein kinase is provided,
b] at least one substrate molecule of the protein kinase of a] is provided,
c] at least one donor component which is able to bind to the substrate molecule from b] and which emits a signal after the donor component was irradiated by a light source,
d] at least one acceptor component which is able to recognize the substrate of b] in case it had been phosphorylated and which acceptor component is able to receive a signal in the form of an electromagnetic radiation is provided,
e] the protein kinase from a], the substrate molecule from b], the donor component from c] and the acceptor component from d] are brought in contact,
f] the mixture of e] is irradiated with a light source in such a way that the donor component of c] is excited,
g] the electromagnetic radiation which is emitted by the mixture of e] is detected.

A substrate molecule shall mean any chemical compound which is phosphorylatable by a protein kinase. A substrate molecule consists preferably of a peptide or a protein. A substrate molecule may consist of a G-Protein coupled Receptor or a member of a signaling cascade.

A donor component is able by means of a donor compound to emit a signal after irradiation by a light source. The signal may consist of an electromagnetic radiation which is characterized by a specific wavelength or may consist of an atom or of a molecule or another type of output.

An acceptor component is able to emit electromagnetic radiation upon having received the signal from the donor component. This electromagnetic radiation is characterized by its specific wavelength. It can be detected by means of an analysis apparatus as e.g. by a spectrometer.

In further embodiments of the invention the substrate molecule can be linked to an antibody which is able to bind to the donor component or the donor component is linked to an antibody which is able to bind to the substrate molecule.

In a further preferred embodiment of the invention the substrate molecule can be linked to a first antibody and the donor component can be linked to a second antibody wherein either the first antibody is able to bind to the second antibody or the second antibody is able to bind to the first antibody.

The linking of a compound to one or several other compounds shall mean that the connection between these two compounds occurs permanently. The linking is realized e.g. by covalent chemical bondages, or electrostatic forces as mediated by ionized molecules or by other means.

The binding of a compound to one or several other compounds shall start from a non-binding stage, then continue to the contracting of the two compounds after finally forming a strong bondage between the said compounds. This bondage may be characterized by binding constants which are preferably within range of $5 \times 10^{-9}$ to $5 \cdot 10^{-7}$M.

In a further embodiment of the method the substrate component is linked to a biotin molecule and the donor component is linked to streptavidin or the substrate component is linked to streptavidin and the donor compound is linked to biotin.

Biotin and streptavidin can bind to each other. Other chemical groups linked to the substrate component or donor component which are able to bind to each other comprise e.g. DNA and RNA binding proteins and the corresponding polynucleotide binding motifs.

The Protein kinase is e.g. the beta-Adrenerge-Receptor-Kinase1 or the G-Protein-Coupled-Receptor-Kinase2.

The substrate can consist of a G-protein coupled Receptor or of the muscarinic Acetylcholine-Receptor or an adrenergic alpha 2 Receptor.

In a further embodiment of the invention the acceptor component is linked to at least one antibody which specifically binds to the substrate of b] in case this substrate had been phosphorylated. Such an antibody is e.g. the monoclonal antibody E11/19. Manufacturing of this antibody was described in "Pollak-Kapp. et al., J. Biol. Chem. (2003), 278, 2190-2198".

The electromagnetic radiation which is emitted by the mixture according to g] of the method of the invention for detecting the phosphorylation of a substrate molecule can be fluorescence radiation. The electromagnetic radiation according to g] may be detected, for example, with the aid of a photomultiplier.

The light source for irradiation of the mixture according to f] of the method of the invention for detecting the phosphorylation of a substrate molecule can be a laser or a lamp.

The signal from the donor component to the acceptor component can be transferred preferably by emissionless energy transfer. This energy transfer can be a fluorescence resonance energy transfer.

In this case it is possible to carry out the method, for example, in such a way that the at least one compound or group of compounds according to b] (donor group) comprises a europium salt-containing compound and that the at least one compound or group of compounds according to c] (acceptor group) may comprise allophycocyanine (Grepin et al. (2000), Drug Discovery Today 5, 212).

Alternatively, the donor and acceptor groups may be dyes suitable for emissionless energy transfer. It is furthermore possible to use all compounds known to the skilled worker which are suitable for emissionless energy transfer, in particular for fluorescence resonance energy transfer (see, for example, Pope et al. (1999), Drug Discovery Today 4, 350). In this embodiment, preference is given to choosing the binding components to be used in such a way that the distance between the donor and acceptor groups after binding is less than 10 nm, since an effective emissionless energy transfer is probably not possible at a larger distance.

The signal from the donor component to the acceptor component can be transferred via singlet oxygen.

In a further embodiment of the method of the invention for detecting the phosphorylation of a substrate molecule the donor component according to c] can comprise a compound which is able to convert triplet oxygen to singlet oxygen after excitation by a laser wherein the acceptor component according to d] comprises at least one first compound excitable by singlet oxygen and at least one second compound capable of absorbing in an emissionless manner and emitting in the form of fluorescence radiation the energy absorbed by the first compound.

The singlet oxygen formed may diffuse from the donor group to the acceptor group and react with the chemiluminescent substances present there. The energy released in the process is transferred to the fluorophores which finally emit the energy in the form of fluorescence radiation which can be detected using a photomultiplier. A precondition for a detectable signal is the spatial proximity of donor and acceptor beads, since singlet oxygen is unstable and decays in aqueous solution. Therefore, the binding components to be used in this method are chosen in such a way that, when the binding event occurs, the distance between the donor and acceptor groups is preferably less than 200 nm.

Particular preference is given in this embodiment to donor group and acceptor group localized on particles, the particles preferably having an average diameter of between 150 and 250 nm, particularly preferably of approximately 200 nm. The preferred use of the particles here is such that the final concentration of acceptor group-carrying particles is 1-40 µg/ml and the final concentration of acceptor group-carrying particles is 1-80 µg/ml. The binding capacity of the particles may be, for example, from approximately 0.1 nM to 1 nM per µm/ml particles.

Particular preference is given to using AlphaScreen beads from Perkin-Elmer Life Sciences as particles in the method. In this embodiment, the donor group is excited by irradiating at a wavelength of 680 nm, using a laser, and the radiation emitted by the acceptor group can be detected at a wavelength between 520 and 600 nm. According, it is possible, in this case, for the method to be carried out in such a way that donor and acceptor beads carrying donor and acceptor groups are introduced initially.

The donor and/or acceptor compound can consist of particles on which donor groups or acceptor groups are localized on. Such particles have preferably an average diameter of approximately 200 nm.

The method of the invention for detecting the phosphorylation of a substrate molecule may further comprise in another embodiment the steps of adding at least one test substance and observing how the test substance influences the activity of the protein kinase.

The method of the invention is in either case an HTS (high throughput screening)-capable method which may be carried out homogenously in the form of a one-step method. In a preferred embodiment, the method of the invertion is carried out according to the "mix and measure" principle.

The HTS-capable assay systems of the invention are used here preferably for carrying out the above-described methods of the invention.

A particular characteristic feature of the methods of the invention is the possibility of monitoring the kinasing of the substrate in solution, since none of the reactants needs to be immobilized on a solid phase. Moreover, it is possible to measure the protein kinases from biological sources directly.

One may employ a reduced sample volume of the method of the invention compared to convential methods. Thus it is possible to use volumes of less than 10 µl. Moreover, it is possible to use highly diluted solutions of the substrate of less than 1 nM, even less than 100 pM.

Fluorescence is, in theory, the most sensitive analytical technique. As many intra- and intermolecular processes are able to modulate the label emission, it was therefore under consideration very early on as a technique of choice for designing homogeneous assays. Polarization, quenching, time correlation, lifetime variation as well as fluorescence resonance energy transfer (FRET) have been applied to probing molecular interactions. Among these techniques, FRET is of particular interest.

However, severe limitations still remain for most of these applications, including low modulation range, interference from the assay medium (serum, biological fluid others), non specific interaction with media constituents, highly variable fluorescence background and light scattering from proteins, molecules and aggregates in the media.

For commonly known donor-acceptor pairs, the distance $R_0$ (for which transfer is 50% efficient) lies within the 1-7 nm range. Taking advantage of these properties, FRET was used as a spectroscopic ruler to reveal proximity relationships in biological macromolecules.

Since only a very few techniques allow such distance and interactions to be determined in solution, many domains of biology were explored with FRET, such as enzymatic activity, protein-DNA interactions, and cell surface lectin revealed receptor-ligand interactions. DNA hybridization has attracted much attention and is now becoming a field of investigation for FRET experiments, either for structural investigations, olinucleotide hybridizations or detection of gene translocation. Because the processes involved in FRET occur within distances characteristic of antigen antibody interactions, homogeneous fluoroimmunoassays have also been developed. More recently, FRET was used to monitor receptor oligomerisation, a key process in the cell signaling pathway.

In one embodiment the invention comprises an assay for determining the activity of a G-Protein-Coupled-Receptor specific protein kinase wherein

a] a protein kinase is provided,
b] a donor bead, which streptavidin is linked to, of an amplified luminescent proximity homogenous assay is provided,
c] a substrate molecule, which a biotin molecule is covalently linked to, of the protein kinase from a] is provided, which substrate molecule is phosphorylatable by the protein kinase from a],
d] an antibody is provided that specifically recognises the phosphorylated substrate molecule from c]
e] an acceptor bead, which is or can be linked to the antibody from d] by means of, e.g., protein A or a suitable secondary antibody, of an amplified luminescent proximity homogenous assay is provided,
f] the protein kinase from a], the donor bead from b], the substrate molecule from c], the antibody from d] and the acceptor bead from e] are incubated simultaneously,
g] the emission of light is determined at one or several wave lengths between 520 and 620mm,
h] the activity of the protein kinase from a] is calculated from emission of light accordingly to g] wherein the maximum emission of light shall indicate the standard activity of the protein kinase,
i] possibly the activity of a second protein kinase, which is of the same type of species as the protein kinase from a] or which is of a different type of species as the protein kinase from a], is determined and calculated accordingly to steps a] to h] wherein the calculation accordingly to h] for the protein kinase from a] is taken as reference for comparing the activity of the second protein kinase thereby indicating a decrease of activity of the second protein kinase in case the emission of light of the second protein kinase is diminished when compared to the emission of light of the protein kinase from a].

In preferred embodiments of the assay for determining the activity of a G-Protein-Coupled-Receptor specific kinase as mentioned before the protein kinase according to a] is selected from the group of β-Adrenergic-Receptor-Kinase 1 (βARK1) or G-Protein-Coupled-Receptor 2 (GRK2).

In further embodiments of the assay for determining the activity of a G-Protein-Coupled-Receptor specific kinase as mentioned before the substrate according to c] consists preferably of a G-Protein-Coupled-Receptor which is selected from the group of a muscarinic Acetylcholine-Receptor or an adrenergic alpha 2 Receptor.

In additional embodiments of the assay for determining the activity of a G-Protein-Coupled-Receptor specific kinase as mentioned before the substrate according to c] consists preferably of a peptide being composed of between 10 and 50 amino acids or consists more preferably of a peptide being composed of an amino acid sequence of Seq. ID No. 1.

In a further preferred embodiment of the assay for determining the activity of a G-Protein-Coupled-Receptor specific kinase as mentioned before as antibody according to d] the monoclonal antibody E11/19can be used. Manufacturing of such antibody was described in "Pollok-Kopp et al., J. Biol. Chem. 2003, 278, 2190-2198". Such antibody can be linked to the acceptor bead via protein A or a suitable secondary antibody.

In one embodiment the invention refers to a kit of parts, which kit of parts comprises

a] at least one protein kinase,
b] at least one donor bead, which streptavidin is linked to, of an amplified luminescent proximity homogenous assay,
c] at least one substrate molecule, which a biotin molecule is covalently linked to, of a protein kinase from a] which substrate molecule is phosphorylatable by a protein kinase from a],
d] an antibody is provided that specifically recognises the phosphorylated substrate molecule from c]
e] at least one acceptor bead, which is or can be linked to the antibody from d] by means of, e.g., protein A or a suitable secondary antibody, of an amplified luminescent proximity homogenous assay is provided.

The components of the kit of parts may be used simultaneously or one after another in one combined or several distinguished use forms. A use form consists for example of an incubation approach including a vessel for incubation, a liquid for incubation and incubation conditions as temperature or time.

In preferred embodiments of the kit of parts as mentioned before the protein kinase according to a] is selected from the group of β-Adrenergic-Receptor-Kinase1 (βARK1] or G-Protein-Coupled-Receptor-Kinase2 (GRK2].

In further embodiments of the kit of parts as mentioned before the substrate according to c] consists preferably of a G-Protein-Coupled-Receptor or more preferably of a G-Protein-Coupled-Receptor which is selected from the group of a muscarinic Acetylcholine-Receptor or an adrenergic alpha2 Receptor.

In additional embodiments of the kit of parts as mentioned before the substrate according to c] consists preferably of a peptide being composed of between 10 and 50 amino acids or consists more preferably of a peptide being composed of an amino acid sequence of Seq. ID No. 1.

In further preferred embodiment of a kit of parts as mentioned before as antibody according to d] the monoclonal antibody E11/19can be used. Such antibody can be linked to the acceptor bead via protein A or a suitable secondary antibody.

In one embodiment the invention comprises the manufacturing of a kit of parts which kit of parts has been described as mentioned before wherein

a] at least one protein kinase is provided,

b] at least one donor bead, which streptavidin is linked to, of an amplified luminescent proximity homogenous assay is provided,

c] at least one substrate molecule, which a biotin molecule is covalently linked to, of a protein kinase from a] which substrate molecule is phosphorylatable by a protein kinase from a], is provided,

d] an antibody is provided that specifically recognises the phosphorylated substrate molecule from c]

e] at least one acceptor bead, which is or can be linked to the antibody from d] by means of, e.g., protein A or a suitable secondary antibody, of an amplified luminescent proximity homogenous assay is provided,

f] the components from a] to e] are packaged individually or combined in one or several containers,

g] at least one container from f] is possibly packaged into at least one second container,

h] at least one container from f] and/or g] is supplied with instructive material and/or labeled.

The kit of parts as described before can be used for determining the activity of a protein kinase and/or can be used in a screening process which may be organized as High-Throughput-Screening (HTS) for identifying a compound which increases the activity of a protein kinase and/or can be used for identifying a compound which decreases the activity of a protein kinase and/or can be used in a screening process which may be organized as High-Throughput-Screening (HTS] for identifying a compound which decreases the activity of a protein kinase.

In one embodiment the invention comprises a method for identifying a compound, which decreases the activity of a G-Protein-Coupled-Receptor specific protein kinase, wherein

a] a protein kinase is provided,

b] a donor bead, which streptavidin is linked to, of an amplified luminescent proximity homogenous assay is provided,

c] a substrate molecule, which a biotin molecule is covalently linked to, of the protein kinase from a] is provided, which substrate molecule is phosphorylatable by the protein kinase from a]

d] an antibody is provided that specifically recognises the phosphorylated substrate molecule from c]

e] an acceptor bead, which is or can be linked to the antibody from d] by means of, e.g., protein A or a suitable secondary antibody, of an amplified luminescent proximity homogenous assay is provided,

f] a chemical compound is provided,

g] the protein kinase from a], the donor bead from b], the substrate molecule from c], the antibody from d], the acceptor bead from e] and the chemical compound from f] are incubated simultaneously,

h] the emission of the light is determined at one or several wave lengths between 520 and 620 nm,

i] the activity of the protein kinase from a] is calculated from emission of light accordingly to h] wherein the maximum emission of light shall indicate the standard activity of the protein kinase,

j] the activity of the protein kinase as calculated accordingly to i] is set in relation to the result of a test wherein the protein kinase from a], the donor bead from b], the substrate molecule from c], the antibody from d] and the acceptor bead from e] are incubated simultaneously thereby indicating a decrease of the protein kinase if the activity of the protein kinase is diminished in case the protein kinase is incubated in presence of the chemical compound accordingly to f].

In preferred embodiments of a method for identifying a compound which decreases the activity of a G-Protein-Coupled-Receptor specific protein kinase as mentioned before the protein kinase according to a] is selected from the group of β-Adrenergic-Receptor-Kinase1 (βARK1] or G-Protein-Coupled-Receptor-Kinase2 (GRK2].

In further embodiments of a method for identifying a compound which decreases the activity of a G-Protein-Coupled-Receptor specific protein kinase as mentioned before the substrate according to c] consists preferably of a G-Protein-Coupled-Receptor or consists more preferably of a G-Protein-Coupled-Receptor which is selected from the group of a muscarinic Acetylcholine-Receptor or an adrenergic alpha 2-Receptor.

In additional embodiments of a method for identifying a compound, which decreases the activity a G-Protein-Coupled-Receptor specific protein kinase as mentioned before the substrate according to c] consists preferably of a peptide being composed of between 10 and 50 amino acids or consists more preferably of a peptide being composed of an amino acid sequence of Seq. ID No. 1.

In a further preferred embodiment of a method for identifying a compound, which decreases the activity of a G-Protein-Coupled-Receptor specific protein kinase as mentioned before as antibody according to d] the monoclonal antibody E11/19 can be used. Such antibody can be linked to the acceptor bead via protein A or a suitable secondary antibody.

In one embodiment the invention comprises a chemical compound having the ability to diminish the activity of a G-Protein-Coupled-Receptor specific kinase which is identified by a method for identifying a compound, which decreases the activity of a G-Protein-Coupled-Receptor as has been described in a former part of this invention. Such compound has preferably a mass of between 0,1 to 50 kDa. Such compound is preferably selected from one of the following groups of a protein, an amino acid, a polysaccharide, a sugar, a polynucleotide, a nucleotide, a fatty acid containing compound, a fat, a fatty acid, a derivative of a fatty acid and or an aromatic carbon compound. Such a compound could be the component of a medicament besides pharmaceutically acceptable expedients for the treatment of a disease which is linked to a disabled phosphorylation of a G-Protein-Coupled-Receptor.

The invention comprises in one embodiment a peptide consisting of an amino acid sequence of Seq. ID No. 1. Such a peptide could be biotinylated. Synthesis of the peptide according to Seq. ID No. 1 carrying or lacking a Biotin-residue can be carried out by a peptide synthesis machine. Such synthesis machines are commercially available e.g. by Perkin Elmer.

The peptide according to Seq. ID No. 1 carrying or lacking a Biotin residue can be used as substrate molecule in a protein kinase test.

The invention concerns further the use of antibody E11/19 for detecting of a protein or peptide which is phosphorylated. This protein or peptide may encompass or consist of an amino acid according to Seq ID No. 1.

The following chapters deal with general technical aspects of the invention.

Providing of a protein can be performed by a several step approach possibly including cloning of the corresponding gene of the protein in an appropriate expression vector, the transformation of e.g. bacterial cells, cultivation of such cells and expression of the said protein, harvesting of the cells from cultivation medium and the breakdown of the cells, and further isolation of the protein by e.g. ion exchange chromatography, size exclusion chromatography, high pressure liquid chromatography, affinity chromatography, polyacrylamide chromatography or other methods. The person skilled in the art will find appropriate methods for purification and handling of proteins in standard textbooks as in e.g. "Current Protocols in Protein Science - ISBN:0-471-11184-8 (eds: John E. Coligan, Ben M. Duan, Hidde L. Ploegh, David W. Speicher, Paul T. Wingfield; Wiley Interscience 1995-2003)"

Providing a cell includes the preparation, cultivation and further processing of the cell. Cells are provided, for example, by preparing suitable cell material from organs or tissues or by propagating suitable cell lines or microorganisms. Various suitable culture media can be used for cultivation. The cells are maintained at the optimum temperature for the organism. Where appropriate, preservatives, antibiotics, pH indicators, blood serum components, blood serum, auxiliaries or the other substances are added to the growth medium used in each case. Processes for preparation, cultivation and further processing are described in standard textbooks (Example: Basic Cell Culture; Ed. J.M. Davis; IRL Press; 1994).

The application of recombinant techniques provides e.g. for a construct to be expressed in a cell. Such a construct consisting of a polynucleotide sequence can be prepared by a skilled worker in a routine manner with the aid of his specialist knowledge. The worker skilled in molecular biology / biochemistry can find the specialist knowledge for this, for example, in "F.M. Ausubel et al.; Current Protocols in Molecular Biology; John Willey & Sons; New York". A vector construct for example is prepared by incorporating a polynucleotide coding for the amino acid sequence of, for example, a GPCR into an expression vector. An expression vector is a vector in which a polynucleotide sequence can be translated in a host cell into a protein. Vectors may be derived from plasmids, viruses or cosmids and must be capable of autonomous replication. They generally contain an origin of replication, cleavage sites for restriction enzymes and marker genes such as, for example, antibiotic resistance genes. In an expression vector, the polynucleotide sequence which is to be propagated or which has been introduced from the outside is under the functional control of a promoter. A promoter is a functional polynucleotide sequence of variable length, which is used to control transcription, i.e. synthesis of mRNA of a polynucleotide sequence immediately 3'- of said promoter. There are promoters which are active only in prokaryotes, such as, for example, the lac, tac and trc promoters, and also promoters which are active only in eucaryotes, such as, for example, CMV or ADH promoters. In a preferred embodiment, the recombinant vector construct comprises an expression vector usable in eucaryotes and/or procaryotes. An expression vector contains a promoter which can be linked functionally to a polynucleotide sequence so that a protein encoded by said polynucleotide sequence is synthesized in an organism, for example a bacterium, fungus or the cell of a eucaryotic cell line. The promoter may be inducible, e.g. by means of tryptophan, or may be constitutively active. Examples of expression vectors are pUC18, pUC19, pBluescript, pcDNA3.1.

Transfection is the introduction of foreign polynucleotide sequences into a host cell and the subsequent propagation of said polynucleotide sequence to any number of identical copies.

A cell line is transiently transfected with a recombinant construct by means of routine methods which can be found by the skilled worker in the above mentioned textbook "Current Protocols in Molecular Biology, published by John Wiley & Sons, New York", or in "Sambrook et al.; A Laboratory Manual, Cold Spring Harbor Laboratory, ISBN 0-87969-309-6". Examples of such routine methods are electroporation, $Ca^{2+}$- phosphate coprecipitation and transfection by means of liposomes. The transfected genes may be expressed in the host cell by Western blotting of cell lysates of transfected cells in combination with an immunological detection method. For this too, the required laboratory protocols can be found by the skilled worker in the manuals mentioned above. Specific antibodies for immunodetection of different GPCR receptors are commercially available.

A chemical compound is provided in particular by chemical synthesis or isolation of chemical substances from biological material.

The skilled worker may use routine methods for chemical synthesis of a compound or isolation of a substance from cell. Such methods are available to the skilled worker in textbooks such as "Organic Synthesis Workbook; 1995; John Wiley & Sons; ISBN 3-527-30187-9", "The Organic Chemistry of Drug Synthesis; 1998; John Wiley & Sons; ISBN 0-471-24510-0", or "Bioactive Compounds from Natural Sources; 2001; Taylor & Francis; ISBN 0-7484-0890-8".

The compounds obtained by synthesis or isolation may be dissolved in a suitable solvent. Suitable solvents may contain water, buffer substances (e.g. Tris, HEPES, MOPS, etc.], monovalent and/or divalent ions (e.g. $K^+$, $Na^+$, $Mg^{2+}$, $Ca^{2+}$, etc.], acids (e.g. HCl, $H_2SO_4$, formic acid, acetic acid, etc.], bases (e.g. NaOH, etc.], alcohol (e.g. methanol, ethanol, glycerol], detergents (e.g. Na dodecyl sulfate, etc.], organic solvents (e.g. formamide, acetone, dimethyl sulfoxide, etc.] and other components, in particular solubilizers and stabilizers.

The skilled worker is able to bring in contact the components of the assay of the invention by using laboratory routine methods. Contacting may take place, for example, in Erlenmeyer vessels, tubes, Eppendorf vessels or in microtiter plates. Temperature-controlled incubators for which a constant temperature of, for example, 30°C or 37°C and fixed $CO_2$ or humidity conditions can be set may be used for said contacting. Contacting may in particular also be carried out in laboratory robot devices. Contacting is possible for different periods of time, from a few seconds to minutes and up to several hours. The conditions to be chosen in each case depend on the receptor, the cell line and the chemical compound.

The final form of pharmaceutical relates to the final formulation, for example, as tablet, granules, spray, solution, ointment, tincture or other formulation forms.

Processing to the final form refers to the preparation of the particular formulation in generally, the daily dose is in the range from 0.3 mg to 100 mg (typically from 3 mg to 50 mg) per day and per Kilogram of body weight, for example, 3-10 mg/kg/day. An intravenous dose may be, for example, in the range from 0.3 mg to 1.0 mg/kg and can most suitably be administered as infusion of from 10 ng to 100 ng per kilogram and per minute. Suitable infusion solutions for these purposes may contain, for example, from 0.1 ng to 10 mg, typically from 1 ng to 10 mg, per milliliter. Single doses may contain, for example, from 1 mg to 10 g of the active substance. It is thus possible for ampoules for injections to contain, for example, from 1 mg to 100 mg, and for single-dose formulations which can be administered orally, such as, for example, tablets or capsules, to contain, for example, from 1.0 to 1000 mg, typically from 10 to 600 mg.

Examples:

Materials and methods with respect to the forthcoming experimental protocols have been summarized in tables 1, 2 and 3.

Identification of AlphaScreen artifacts:

Compounds: 100 µM in test buffer A

Detection solution: anti-streptavidin antibody (1:833], streptavidin donor beads (20 µg/ml], protein-A acceptor beads (40 µg/ml] in a buffer mixed from 1 part test buffer A containing the kinase, 2 test buffer A containing the kinase substrates and 2 parts test buffer B containing the phosphospecific antibody

- - 1 µl compound
- - + 5 µl detection solution
- - seal the plate, incubate overnight at room temperature
- - read the plate in the Alpha Quest

Dilution of compounds:

(1) For single concentration screening:

- • dilute 1 µl compound (10 mM in dimethylsulfoxide) with 100 µM test buffer A

(2) For dose-response measurements:

- - 5 µl 10 mM in DMSO (Dimethylsulfoxide]
- - + 120 µl 20 mM Tris-HCl, pH 7.4, 20 mM MgCl$_2$, 4 mM EDTA, 0.2 % BSA, 5 % DMSO (400 µM, corresponds to 100 µM in enzymatic reaction)
- - serial 1+1 dilution (40 + 40 µl] in 20 mM Tris-HCl, pH 7.4, 20 mM MgCl$_2$, 4 mM EDTA, 0.2 % BSA, 9 % DMSO, 7 dilutions (overall concentration range 400-3.125 µM, [DMSO]=9 % at all compound concentrations]

Reaction:

- - 1 µl compound
- - +1 µl reaction mix A without DMSO ([DMSO]=2.25 % during reaction)
- - + 2 µl ATP, incubate for 120 min at room temperature
- - + 2 µl stop/detection solution, seal the plate, incubate over night at room temperature
- - read the plate

Data analysis

Calculation of percent-inhibition values
Percent-inhibition values are calculated as:

$$\%\text{inhibition} = 100 \cdot \left[ \frac{\text{value} - \text{low control}}{\text{high control} - \text{low control}} \right]$$

Calculation of assay quality parameters

Assay quality parameters were determined on the basis of high and low controls, which were run as internal standards on each assay plate. The following quality parameters are used as a measure to judge the suitability of an assay for high throughput screening.
Signal to background (S/B)

$$\text{S/B} = \frac{\text{mean}_{\text{high control}}}{\text{mean}_{\text{low control}}}$$

Z'-factor

$$Z' = 1 - \frac{(3 * SD_{\text{high control}} + 3* SD_{\text{low control}}]}{|\text{mean}_{\text{high control}} - \text{mean}_{\text{low control}}|}$$

Identification of positives

Wells with %inhibition values larger than (mean+3σ] were considered as positives.

Purification of GRK

Several methods for purification of GRK are available from the state of the art. In "Söhlemann et al., 1993, FEBS Lett. 324, 59-62" the purification and functional characterization of β-adrenergic receptor kinase after having expressed the kinase in insect cells was described.

Furthermore, "Kim et al., 1993, Receptor 3, 39-55" described expression and characterization of two β-adrenergic receptor kinase isoforms in a baculovirus expression system.

Manufacturing of antibodies

The construction of hybridoma cell lines producing monoclonal antibodies against different GRK derived peptides has been described in amongst others "Martin Oppermann et al., 1999, Journal of Biol. 274, 8875-8895".

Manufacturing of murine monoclonal antibody in particular of E11/19 (or others as R22/7, T21/2) which either binds to a CCR5 N-terminal epitope and in particular to an peptide having an amino acid sequence according to SEQ ID NO. 1 was described in "Pollok-Kopp et al., J. Biol. Chem. 2003, 278, 2190-2198".

Description of tables

Table 1:     Stock solutions
Table 2:     Buffers and reagents
Table 3:     Reagent Concentrations in the assay
Table 4:     Summary of results of the primary screen of two test sets

Table 1:

| name | composition | preparation | storage |
|---|---|---|---|
| Tris buffer A | 20 mM, pH 7.4 | dissolve 2.4 g Tris in $H_2O$, adjust pH with HCl, make up to 1 l with $H_2O$ | room temperature |
| Tris buffer B | 10 mM, pH 7.4 | dissolve 1.2 g Tris in $H_2O$, adjust pH with HCl, make up to 1 l with $H_2O$ | room temperature |
| EDTA_A | 0.5 M, pH 7.4 | dissolve 190 g EDTA in $H_2O$, adjust pH to 7.4 with NaOH, make up to 1 l with $H_2O$ | 4 °C |
| EDTA_B | 0.5 M, pH 7.8 | dissolve 190 g EDTA in $H_2O$, adjust pH to 7.8 with NaOH, make up to 1 l with $H_2O$ | 4 °C |
| BSA | 10 % | dissolve 100 g BSA in 1 l $H_2O$ | -20 °C |
| ATP | 400 µM | dissolve 220 mg ATP in 1 l $H_2O$ | -20 °C |
| $MgCl_2$ | 2.4 M | add 228 g $MgCl_2$ to 1 l $H_2O$ | 4 °C |
| CCR5 peptide | 350 µM in DMSO | dissolve 0.98 mg in 1 ml DMSO | -20 °C |
| CCR5 peptide | 3.5 µM in Tris buffer A | dilute CCR5 DMSO stock 1:100 in Tris buffer A | -20 °C (aliquots] |
| βARK | 12.9 mg/ml (162 µM] | use as provided by protein production | -80 °C |

**EP 1 514 944 A1**

Table 1:   (continued)

| name | composition | preparation | storage |
|---|---|---|---|
| Anti-CCR5 | 1,22 mg/ml (7.4 µM] | use as provided by Martin Oppermann (University of Göttingen] | 4 °C |
| Heparin | 300 µM | provided by J. Huber, prepared by dissolving 5.1 mg in 1 ml 20 mM Hepes-KOH, pH 7.4, 2 mM EDTA, 1 % DMSO | -20 °C |
| wash solution A | 10 % ethanol | mix 100 ml ethanol and 900 ml Tris buffer A | room temperature |
| wash solution B | 10 % ethanol, 0.001 % pluronic | mix 100 ml ethanol, 900 ml $H_2O$ and 0.1 ml 10 % Pluronic F-68 | room temperature |

Table 2:

| name | preparation | stability |
|---|---|---|
| test buffer A | 1 liter: 932 ml Tris buffer A, 8 ml EDTA_A, 20 ml BSA, 40 ml DMSO | one day |
| test buffer B | 1 liter: 986 ml Tris buffer B, 4 ml EDTA_A, 10 ml BSA | one day |
| compounds | add 100 µl test buffer A to 1 µl compound | one day |
| heparin | dilute 1 µl 300 µM stock in 749 µl test buffer A | one day |
| reaction mix A | 3 ml: 345 µl CCR5 peptide (3.5 µM], 37 µl □□RK, 50 µl $MgCl_2$, 2568 µl test buffer A | 60 min |
| reaction mix B | 3 ml: 345 µl CCR5 peptide (3.5 µM], 50 µl $MgCl_2$, 2568 µl test buffer A | 60 min |
| ATP | dilute stock 1:20 in $H_2O$ | one day |
| stop/detection solution | 5 ml: 4061 µl test buffer B, 60.2 µl donor beads (5 mg/ml], 120.5 µl acceptor beads (5 mg/ml], 150 µl BSA, 600 µl EDTA_B, 8.1 µl anti-pCCR5 | 60 min (store in the dark] |

Table 3:

| component | concentration during reaction | concentration during detection |
|---|---|---|
| buffer | 10 mM Tris-HCl, 10 mM $MgCl_2$, 2 mM EDTA, 0.1 % BSA, pH 7.4 | 10 mM Tris-HCl, 10 mM $MgCl_2$, 22 mM EDTA, 0.1 % BSA, pH 7.8 |
| DMSO | 2.25 % | 1.125 % |
| □ARK | 0.5 µM | 0.33 µM |
| CCR5 peptide | 100 nM | 67 nM |
| ATP | 10 µM | 6.7 µM |
| anti-pCCR5 | - | 4 nM |
| donor beads | - | 20 µg/ml |
| acceptor beads | - | 40 µg/ml |

Table 4:

| Test set | | 020813 | 020819 |
|---|---|---|---|
| | [compound]/μM | 25 | 25 |
| | No. of plates | 48 | 55 |
| | No. of compounds | 16255 | 18592 |
| | mean % inhibition ± SD* | 15 ± 15.4 | 10 ± 13.5 |
| | Threshold for positives (% inhibition]** | 50 | 40 |
| | Number of positives | 764 | 765 |
| | Number of reordered compounds*** | 1124 | 1071 |
| | S/B (avg. per plate] | 1.60 ± 0.04 | 2.31 ± 0.09 |
| | Z-factor (avg. per plate ± SD] | 0.06 ± 0.22 | 0.43 ± 0.15 |
| | Z'-factor (avg. per plate ± SD] | 0.74 ± 0.07 | 0.85 ± 0.06 |

* for wells between -50 and +100 % inhibition (>98.9 % of all data], after correction for border effects

** Due to the relatively small S/B, the threshold for positives was lowered by 10 % compared to the threshold determined as mean + 3□

*** In addition to the positives, all compounds at well positions with >5 positives (see fig. 8] were also re-ordered for confirmation.

Definition of figures

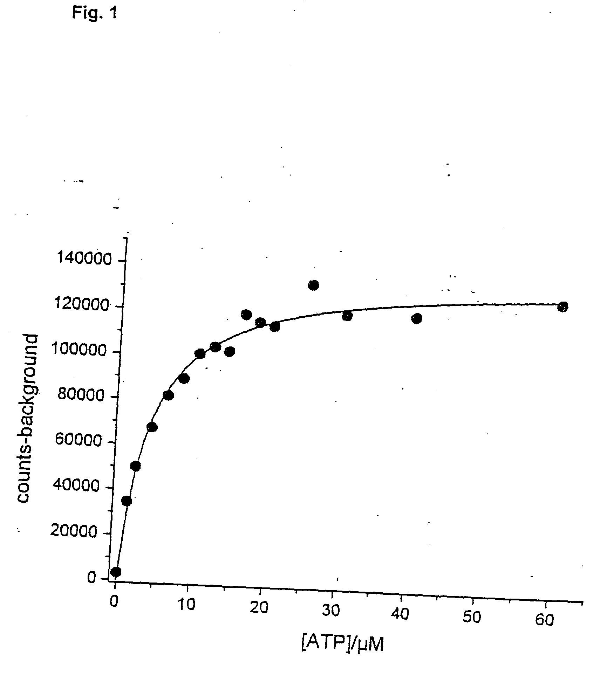

Fig. 1:      Assay validation; ATP dependence; the apparent Km value was determined at 4 μM

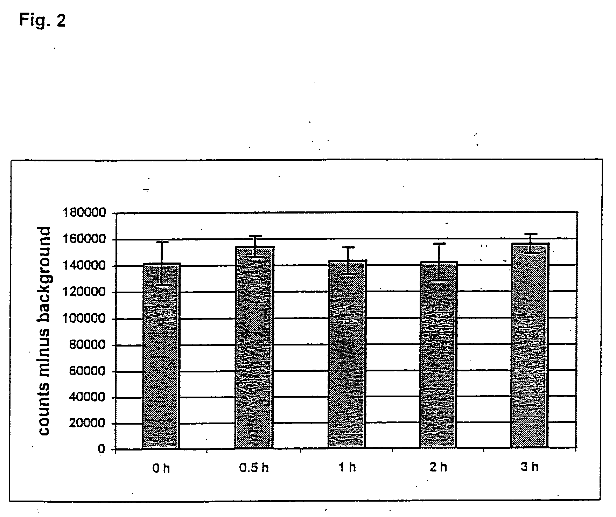

Fig. 2:      Stability of the reaction mix; reagents are stable for more than 3 hours.

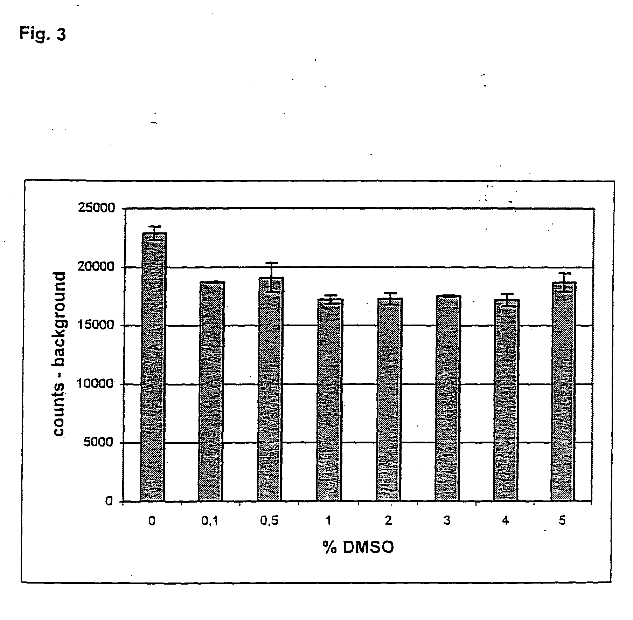

Fig. 3:      DMSO dependence of the assay; the assay tolerates up to 5% DMSO

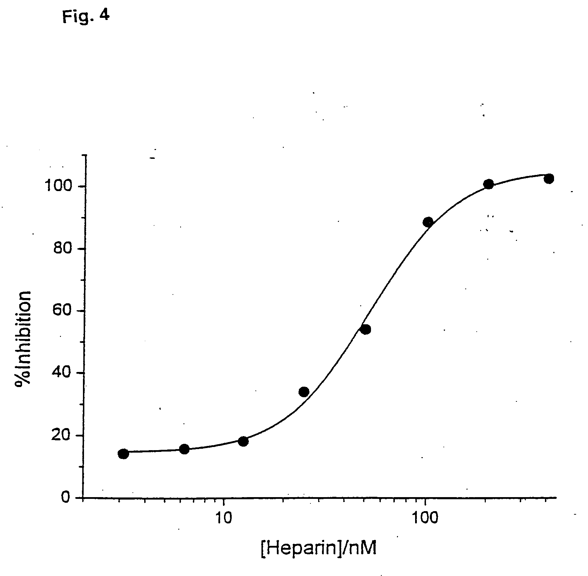

Fig. 4:      Dose-response curve for heparin; the $IC_{50}$ was determined as 50 nM

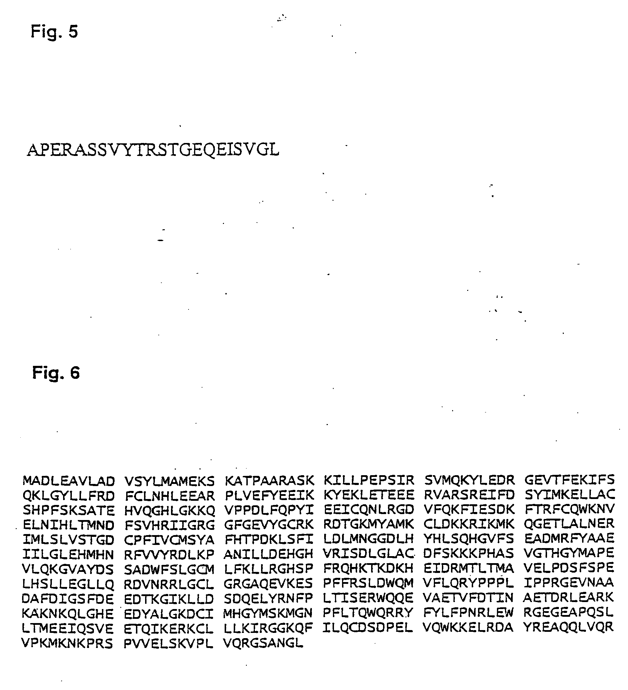

Fig. 5:      Amino acid sequence according to Seq. Id No.1

Fig. 6:      Amino acid sequence including the βARK-1 Sequence according to Seq. ID No. 2

## Claims

1. Method for detecting the phosphorylation of a substrate molecule by a protein kinase wherein

   a] at least one protein kinase is provided,
   b] at least one substrate molecule of the protein kinase of a] is provided,
   c] at least one donor component which is able to bind to the substrate molecule from b] and which emits a signal after the donor component was irradiated by a light source is provided,
   d] at least one acceptor component which is able to bind to the substrate molecule of b] in case it had been phosphorylated and which acceptor component is able to receive a signal from the donor component of c) and emit the signal in the form of an electromagnetic radiation, is provided,
   e] the protein kinase from a], the substrate molecule from b], the donor component from c] and the acceptor component from d] are brought in contact,
   f] the mixture of e] is irradiated with a light source in such a way that the donor component of c] is excited,
   g] the electromagnetic radiation which is emitted by the mixture of e] is detected.

2. Method as claimed in claim 1 wherein the substrate molecule of b] is linked to an antibody which is able to bind to the donor component of c].

3. Method as claimed in claim 1 wherein the donor component of c] is linked to an antibody which is able to bind to the substrate molecule of b].

4. Method as claimed in claim 1 wherein the substrate molecule of b] is linked to a first antibody and the donor component is linked to a second antibody and wherein either the first antibody is able to bind to the second antibody or the second antibody is able to bind to the first antibody.

**5.** Method as claimed in claim 1 wherein the substrate molecule of b] is linked to a biotin molecule and the donor component of c] is linked to streptavidin.

**6.** Method as claimed in claim 1 wherein the substrate molecule of b] is linked to streptavidin and the donor component of c] is linked to biotin.

**7.** Method as claimed in claim 1, wherein the protein kinase according to a] is selected from the group of beta-Adrenergic-Receptor-Kinase 1 or G-Protein-Coupled-Receptor-Kinase 2

**8.** Method as claimed in claim 1, wherein the substrate of b] consists of a G-Protein-Coupled-Receptor.

**9.** Method as claimed in claim 8, wherein the G-Protein-Coupled-Receptor is selected from the group of muscarinic Acetylcholine-Receptor or adrenergic alpha2-Receptor.

**10.** Method as claimed in claim 1, wherein the substrate molecule of b] comprises a peptide of a length between 10 and 50 amino acids.

**11.** Method as claimed in claim 10, wherein the peptide consists of an amino acid sequence of Seq. ID No. 1.

**12.** Method as claimed in claim 1, wherein the acceptor component of d] is comprising or binding to at least one antibody which specifically recognizes the substrate molecule of b] in case this substrate molecule had been phosphorylated.

**13.** Method as claimed in claim 1, wherein the antibody that is part of or binding to the acceptor component of d] is monoclonal antibody E11/19.

**14.** Method as claimed in claim 1, wherein the electromagnetic radiation according to g] is fluorescence radiation.

**15.** Method as claimed in claim 1, wherein the light source according to f] is selected from the group of consisting of a laser or a lamp.

**16.** Method as claimed in claim 1, wherein the signal is transferred from the donor component of c] to the acceptor component of d] by emission less energy transfer.

**17.** Method of claim 16, wherein the emissionless energy transfer is a fluorescence resonance energy transfer.

**18.** Method of claim 1, wherein the signal is transferred from the donor component of c] to the acceptor component of d] via singlet oxygen.

**19.** Method of claim 1, wherein the donor component of c] comprises a compound which is able to convert triplet oxygen to singlet oxygen after excitation by a laser and wherein the acceptor component of d] comprises at least one first compound excitable by singlet oxygen and at least one second compound capable of absorbing in an emissionless manner and emitting in the form of fluorescence radiation the energy absorbed by the first compound.

**20.** Method of claim 19, wherein the donor component of c] and/or the acceptor component of d] consist of particles on which donor groups or acceptor groups are localized on.

**21.** Method of claim 20, wherein the particles have an average diameter of approximately 200nm.

**22.** Method of claim 1, further comprising the steps of adding at least one test substance and observing the influence of the test substance on the activity of the protein kinase.

**23.** Assay for determining the activity of a G-Protein-Coupled-Receptor specific protein kinase wherein

a] a protein kinase is provided
b] a donor bead, which streptavidin is linked to, of an amplified luminescent proximity homogenous assay is provided,
c] a substrate molecule, which a biotin molecule is covalently linked to, of the protein kinase from a] is provided, which substrate molecule is phosphorylatable by the protein kinase from a],

d] an antibody is provided that specifically recognizes the phosphorylated substrate molecule from c],

e] an acceptor bead, which is linked to the antibody from d] or a suitable secondary antibody of an amplified luminescent proximity homogenous assay is provided,

f] the protein kinase from a], the donor bead from b], the substrate molecule from c], the antibody from d] and the acceptor bead from e] are incubated simultaneously,

g] the emission of light is determined at one or several wave lengths between 520 and 620 nm,

h] the activity of the protein kinase from a] is calculated from emission of light accordingly to g] wherein the maximum emission of light indicates the standard activity of the protein kinase,

i] possibly the activity of a second protein kinase which is of the same type of species as the protein kinase from a] or which is of a different type of species as the protein kinase from a], is determined and calculated accordingly to steps a] to h]

wherein the calculation accordingly to h] for the protein kinase from a] is taken as reference for comparing the activity of the second protein kinase thereby indicating a decrease of activity of the second protein kinase in case the emission of light of the second protein kinase is diminished when compared to the emission of light of the protein kinase from a].

24. An assay as claimed in claim 23 wherein the protein kinase according to a] is selected from the group of beta-Adrenergic-Receptor-Kinase 1 or G-Protein-Coupled-Receptor-Kinase 2.

25. An assay as claimed in claim 23 or 24 wherein the substrate according to c] consists of a G-Protein-Coupled-Receptor.

26. An assay as claimed in claim 25 wherein the G-Protein-Coupled-Receptor is selected from the group of muscarinic Acetylcholine-Receptor or adrenergic alpha2-Receptor.

27. An assay as claimed in claim 23 or 24 wherein the substrate consists of a peptide of a length between 10 and 50 amino acids.

28. An assay as claimed in claim 27 wherein the peptide consists of an amino acid sequence of SEQ ID No.1.

29. An assay as claimed in claim 23 wherein as antibody according to d] the monoclonal antibody E11/19 is used.

30. A kit of parts comprising

a] at least one protein kinase,

b] at least one donor bead, which streptavidin is linked to, of an amplified luminescent proximity homogenous assay,

c] at least one substrate molecule, which a biotin molecule is covalently linked to, of a protein kinase from a] which substrate molecule is phosphorylatable by a protein kinase from a],

d] an antibody is provided that specifically recognizes the phosphorylated substrate molecule from c],

e] at least one acceptor bead, which is linked to the antibody from d] or a suitable secondary antibody of an amplified luminescent proximity homogenous assay is provided.

31. A kit of parts as claimed in claim 30 wherein the protein kinase according to a] in selected from the group of beta-Adrenergic-Receptor-Kinase 1 or G-Protein-Coupled-Receptor-Kinase 2.

32. A kit of parts as claimed in claim 30 or 31 wherein the substrate according to c] consists of a G-Protein-Coupled-Receptor.

33. A kit of parts as claimed in claim 32 wherein the G-Protein-Coupled-Receptor is selected from the group if muscarinic Acetylcholine-Receptor or adrenergic alpha2-Receptor.

34. A kit of parts as claimed in claim 30 or 31 wherein the substrate consists of peptide of a length between 10 and 50 amino acids.

35. A kit of parts as claimed in claim 34 wherein the peptide consists of an amino acid Sequence of SEQ ID No.1.

**36.** A kit of parts as claimed in claim 30 wherein as the antibody according to d] the monoclonal antibody E11/19 used.

**37.** Manufacturing of a kit of parts according to one or several of claims 30 to 36 wherein

a] at least one protein kinase is provided,
b] at least one donor bead, which streptavidin is linked to, of an amplified luminescent proximity homogenous assay is provided,
c] at least one substrate molecule, which a biotin molecule is covalently linked to, of a protein kinase from a] which substrate molecule is phosphorylatable by a protein kinase from a], is provided,
d] an antibody is provided that specifically recognizes the phosphorylated substrate molecule from c],
e] at least one acceptor bead, which is linked to the antibody from d] or a suitable secondary antibody of an amplified luminescent proximity homogenous assay is provided,
f] the components from a] to e] are packaged individually or combined in one or several containers,
g] at least one container from f] is possibly packaged into at least one second container,
h] at least one container from e] and/or g] is supplied with instructive material and/or labeled.

**38.** Use of a kit of parts according to claims 30 to 37 for determining of the activity of a protein kinase.

**39.** Use of a kit of parts according to claim 30 and 37 in a screening for identifying a compound which decreases the activity of a protein kinase.

**40.** Use of a kit of parts as claimed in claim 39, wherein the screening is a High-Throughput-Screening.

**41.** Method for identifying a compound, which decreases the activity of a G-Protein-Coupled-Receptor specific protein kinase, wherein

a] a protein kinase is provided,
b] a donor bead, which streptavidin is linked to, of an amplified luminescent proximity homogeneous assay is provided,
c] a substrate molecule, which a biotin molecule is covalently linked to, of the protein kinase from a] is provided, which substrate molecule is phosphorylatable by the protein kinase from a],
d] an acceptor bead, which an antibody is linked to and which antibody specifically recognizes the phosphorylated substrate molecule from c], of an amplified luminescent proximity homogenous assay is provided,
e] a chemical compound is provided,
f] the protein kinase from a], the donor bead from b], the substrate molecule from c], the acceptor bead from d] and the chemical compound from e] are incubated simultaneously,
g] the emission of light is determined at one or several wave lengths between 520 and 620 nm,
h] the activity of the protein kinase from a] is calculated from emission of light accordingly to g] wherein the maximum emission of light indicates the standard activity of the protein kinase,
i] the activity of the protein kinase as calculated accordingly to h] is set in relation to the result of a test wherein the protein kinase from a], the donor bead from b], the substrate molecule from c], and the acceptor bead from d] are incubated simultaneously thereby indicating a decrease of the protein kinase if the activity of the protein kinase is diminished in case the protein kinase is incubated in presence of the chemical compound according to f] when compared to the results of the test in which the protein kinase is incubated in absence of the chemical compound.

**42.** A method for identifying a compound as claimed in claim 41, wherein the protein kinase according to a] is selected from the group beta-Adrenerge-Receptor-Kinase 1 or G-Protein-Coupled-Receptor Kinase 2.

**43.** A method for identifying a compound as claimed in claim 41 or 42, wherein the substrate according to c] consists of a G-Protein-Coupled-Receptor.

**44.** A method for identifying a compound as claimed in claim 43 wherein the G-Protein-Coupled-Receptor is selected from the group of muscarinic Acetylcholin-Receptor or adrenergic alpha 2-Receptor.

**45.** A method for identifying a compound as claimed in claim 41 or 42, wherein the substrate consists of a peptide of a length between 10 and 50 amino acids.

**46.** A method for identifying a compound as claimed in claim 45 wherein the peptide consists of an amino acid Sequence of Seq ID No.1.

**47.** A method for identifying a compound as claimed in claim 41, wherein as antibody according to d] the monoclonal antibody E11/19 is used.

**48.** Chemical compound with ability to diminish the activity of a G-Protein-Coupled-Receptor specific kinase which is identified by a method according to one or several of claims 41 to 47.

**49.** Chemical compound as claimed in claim 48 wherein the mass of the compound is between 0,1 to 50 kDa.

**50.** Chemical compound as claimed in claim 48 or 49, wherein this compound is selected of one of the following groups of a protein, an amino acid, a polysaccharide, a sugar, a polynucleotide, a nucleotide, a fatty acid containing compound, a fat, a fatty acid, a derivative of a fatty acid or an aromatic carbon compound.

**51.** Medicament comprising a compound according to claims 48 to 50 as well as pharmaceutically acceptable expedients for treatment of a disease, which is linked to wrong phosphorylation of a G-Protein-Coupled-Receptor.

**52.** Peptide consisting of an amino acid sequence of Seq ID No. 1.

**53.** Peptide as claimed in claim 52 wherein the peptide is biotinylated.

**54.** Synthesis of a peptide according to claim 52 or 53 by means of a peptide synthesis apparatus.

**55.** Use of a peptide as claimed in claims 52 to 54 as substrate molecule in a protein kinase test.

**56.** Use of antibody E11/19 for detecting of a protein or peptide which is phosphorylated.

**57.** Use of claim 56 wherein the protein or peptide is encompassing an amino acid of Seq ID No. 1.

Fig. 1

Fig. 2

Fig. 3

Fig. 4

Fig. 5

APERASSVYTRSTGEQEISVGL

Fig. 6

```
MADLEAVLAD  VSYLMAMEKS  KATPAARASK  KILLPEPSIR  SVMQKYLEDR  GEVTFEKIFS
QKLGYLLFRD  FCLNHLEEAR  PLVEFYEEIK  KYEKLETEEE  RVARSREIFD  SYIMKELLAC
SHPFSKSATE  HVQGHLGKKQ  VPPDLFQPYI  EEICQNLRGD  VFQKFIESDK  FTRFCQWKNV
ELNIHLTMND  FSVHRIIGRG  GFGEVYGCRK  RDTGKMYAMK  CLDKKRIKMK  QGETLALNER
IMLSLVSTGD  CPFIVCMSYA  FHTPDKLSFI  LDLMNGGDLH  YHLSQHGVFS  EADMRFYAAE
IILGLEHMHN  RFVVYRDLKP  ANILLDEHGH  VRISDLGLAC  DFSKKKPHAS  VGTHGYMAPE
VLQKGVAYDS  SADWFSLGCM  LFKLLRGHSP  FRQHKTKDKH  EIDRMTLTMA  VELPDSFSPE
LHSLLEGLLQ  RDVNRRLGCL  GRGAQEVKES  PFFRSLDWQM  VFLQRYPPPL  IPPRGEVNAA
DAFDIGSFDE  EDTKGIKLLD  SDQELYRNFP  LTISERWQQE  VAETVFDTIN  AETDRLEARK
KAKNKQLGHE  EDYALGKDCI  MHGYMSKMGN  PFLTQWQRRY  FYLFPNRLEW  RGEGEAPQSL
LTMEEIQSVE  ETQIKERKCL  LLKIRGGKQF  ILQCDSDPEL  VQWKKELRDA  YREAQQLVQR
VPKMKNKPRS  PVVELSKVPL  VQRGSANGL
```

European Patent Office

**PARTIAL EUROPEAN SEARCH REPORT**

which under Rule 45 of the European Patent Convention shall be considered, for the purposes of subsequent proceedings, as the European search report

Application Number

EP 03 02 0429

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y,D | POLLOCK-KOPP B. ET AL.: "Analysis of ligand-stimulated CC chemokine receptor 5 (CCR5) phosphorylation in intact cells using phosphosite-specific antibodies. " J. BIOL. CHEM., vol. 278, no. 4, 24 January 2003 (2003-01-24), pages 2190-2198, XP002268045 | 1-47 | C12Q1/48 G01N33/566 |
| X | * the whole document * --- | 52-57 | |
| X | SILLS M.A. ET AL.: "Comparison of assay technologies for a tyrosine kinase assay generates different results in high throughput screening." J. BIOMOL. SCREEN, vol. 7, no. 3, 2002, pages 191-214, XP009024655 * the whole document * --- -/-- | 1-47 | |

TECHNICAL FIELDS SEARCHED (Int.Cl.7)

C12Q
G01N

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 27 January 2004 | Giry, M |

European Patent
Office

INCOMPLETE SEARCH
SHEET C

Application Number

EP 03 02 0429

Claim(s) searched completely:
      1-47, 52-57

Claim(s) not searched:
      48-51

Reason for the limitation of the search:

Present claims 48-51 relate to an extremely large number of possible
compounds. However, no support within the meaning of Article 84 EPC
and/or disclosure within the meaning of Article 83 EPC is to be found in
the description for said compounds. In the present case, the claims so
lack support, and the application so lacks disclosure, that a meaningful
search over the claimed scope is impossible. Consequently, no search has
been carried out for claims 48-51.

European Patent
Office

**PARTIAL EUROPEAN SEARCH REPORT**

Application Number

EP 03 02 0429

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| Y | WU X. ET AL.: "Comparison of assay technologies for a nuclear receptor assay screen reveals differences in the sets of identified functional antagonists." J. BIOMOL. SCREEN., vol. 8, no. 4, August 2003 (2003-08), pages 381-392, XP009024114 * the whole document * --- | 1-47 | |
| Y | ROULEAU N. ET AL.: "Development of a versatile platform for nuclear receptor screening using AlphaScreenTM." J. BIOMOL. SCREEN., vol. 8, no. 2, April 2003 (2003-04), pages 191-197, XP009024113 * the whole document * --- | 1-47 | TECHNICAL FIELDS SEARCHED (Int.Cl.7) |
| Y | WO 02 22882 A (ARCHEMIX CORP.) 21 March 2002 (2002-03-21) * page 29 - page 30 * * page 55-59 * * examples 13,22-26 * --- | 1-47, 52-57 | |
| Y | US 2003/158387 A1 (FORSSMAN W.G. ET AL.) 21 August 2003 (2003-08-21) * paragraph [0190] - paragraph [0191] * * paragraph [0217] - paragraph [0233] * --- | 1-47, 52-57 | |
| Y | BIERI C. ET AL.: "Micropatterned immobilization of a G protein-coupled receptor and direct detection of G protein activation." NATURE BIOTECH., vol. 17, November 1999 (1999-11), pages 1105-1108, XP002183971 * the whole document * --- -/-- | 1-47 | |

**European Patent** **PARTIAL EUROPEAN SEARCH REPORT** **Application Number**

**Office** EP 03 02 0429

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| A | PEPPARD J. ET AL.: "Development of a high-throughput screening assay for inhibitors of aggrecan cleavage using luminescent oxygen channeling (AlphaScreenTM)." J. BIOMOL. SCREEN., vol. 8, no. 2, April 2003 (2003-04), pages 149-156, XP009024115 * the whole document * --- | 1-47, 52-57 | |
| T | LI Y. ET AL.: "Homogeneous assays for adenosine 5'-monophosphate-activated protein kinase." ANAL. CHEM., vol. 321, 15 October 2003 (2003-10-15), pages 151-156, XP004457151 * the whole document * ----- | 1-47, 52-57 | TECHNICAL FIELDS SEARCHED (Int.Cl.7) |

EPO FORM 1503 03.82 (P04C10)

## ANNEX TO THE EUROPEAN SEARCH REPORT
## ON EUROPEAN PATENT APPLICATION NO.

EP 03 02 0429

This annex lists the patent family members relating to the patent documents cited in the above-mentioned European search report.
The members are as contained in the European Patent Office EDP file on
The European Patent Office is in no way liable for these particulars which are merely given for the purpose of information.

27-01-2004

| Patent document cited in search report | | Publication date | Patent family member(s) | | Publication date |
|---|---|---|---|---|---|
| WO 0222882 | A | 21-03-2002 | AU | 9096501 A | 26-03-2002 |
| | | | CA | 2418724 A1 | 21-03-2002 |
| | | | EP | 1354062 A2 | 22-10-2003 |
| | | | WO | 0222882 A2 | 21-03-2002 |
| | | | US | 2003087239 A1 | 08-05-2003 |
| US 2003158387 | A1 | 21-08-2003 | EP | 1167527 A1 | 02-01-2002 |
| | | | AU | 1259501 A | 08-05-2001 |
| | | | CA | 2387378 A1 | 03-05-2001 |
| | | | EP | 1224288 A2 | 24-07-2002 |
| | | | JP | 2003512843 T | 08-04-2003 |
| | | | WO | 0131016 A2 | 03-05-2001 |

EPO FORM P0459

For more details about this annex : see Official Journal of the European Patent Office, No. 12/82